(19) Europäisches Patentamt
European Patent Office
Office européen des brevets

(11) Veröffentlichungsnummer: **0 034 751**
**B1**

(12) **EUROPÄISCHE PATENTSCHRIFT**

(45) Veröffentlichungstag der Patentschrift: **21.11.84**

(51) Int. Cl.³: **A 01 N 43/66,** C 07 D 251/38

(21) Anmeldenummer: **81100922.4**

(22) Anmeldetag: **10.02.81**

(54) **Verfahren zur Herstellung von 1-Amino-1,3,5-triazin-2,4(1H, 3H)-dionen.**

(30) Priorität: **20.02.80 DE 3006263**

(43) Veröffentlichungstag der Anmeldung:
**02.09.81 Patentblatt 81/35**

(45) Bekanntmachung des Hinweises auf die Patenterteilung:
**21.11.84 Patentblatt 84/47**

(84) Benannte Vertragsstaaten:
**AT BE CH DE FR GB IT LI NL SE**

(56) Entgegenhaltungen:
**EP-A-0 034 750**
**DE-A-2 132 936**
**DE-A-2 254 200**
**DE-A-2 311 662**
**DE-A-2 351 556**
**FR-A-1 344 895**
**US-A-3 907 796**

(73) Patentinhaber: **BAYER AG, Konzernverwaltung RP Patentabteilung, D-5090 Leverkusen 1 Bayerwerk (DE)**

(72) Erfinder: **Dickoré, Karlfried, Dr., Nicolai-Hartmann-Strasse 19, D-5090 Leverkusen (DE)**
Erfinder: **Kühle, Engelbert, Dr., von Bodelschwingh-Strasse 42, D-5060 Berg.-Gladbach 2 (DE)**

**EP 0 034 751 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die vorliegende Erfindung betrifft ein neues Verfahren zur Herstellung von teilweise bekannten 1-Amino-1,3,5-triazin-2,4-(1 H, 3 H)-dionen, welche als Herbizide verwendet werden können.

Es ist bereits bekanntgeworden, daß man 1-Amino-1,3,5-triazin-2,4(1 H, 3 H)-dione durch Umsetzung von Imidodicarbonsäure-dichloriden mit Hydrohalogeniden von Isothiosemicarbazonen und anschließende säurekatalysierte Hydrolyse der als Zwischenprodukte zunächst gebildeten 1-Alkylidenamino-Derivate herstellen kann (vgl. DE-OS 2 254 200). Dieses Verfahren weist jedoch eine Reihe von Nachteilen auf. So stellt die Verwendung von Imidodicarbonsäure-dichloriden als Ausgangsstoffe einen erheblichen technischen Aufwand dar, da ihre Herstellung nur nach mehrstufigen Verfahren (vgl. DE-OS 2 351 556) oder über schwer zugängliche Ausgangsprodukte (vgl. DE-OS 1 298 095) möglich ist und außerdem die Ausbeuten nicht befriedigen. Ein weiterer Nachteil des bekannten Verfahrens besteht darin, daß der Ringschluß von Imidodicarbonsäure-dichloriden mit den Hydrohalogeniden von Isothiosemicarbazonen in Gegenwart von drei Molen einer organischen Base in einem organischen Lösungsmittel durchgeführt werden muß. Die technische Durchführung dieses Verfahrens wird durch die erforderliche Rückgewinnung der Lösungsmittel und der Basen zusätzlich erschwert.

Es wurde nun überraschend gefunden, daß man die teilweise bekannten 1-Amino-1,3,5-triazin-2,4-(1 H, 3 H)-dione der allgemeinen Formel

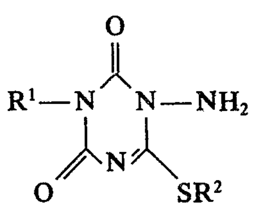

(I)

in welcher

$R^1$ für einen geradkettigen oder verzweigten Alkylrest mit 1—10 C-Atomen, der gegebenenfalls durch $C_{1-4}$-Alkoxy, $C_{1-4}$-Alkylmercapto, Halogen (insbesondere Chlor oder Fluor), Cyano oder Nitro substituiert sein kann; einen Alkenylrest mit 3—8 C-Atomen; einen Alkinylrest mit 3—8 C-Atomen; einen cycloaliphatischen Rest mit 5—8 C-Atomen, der gegebenenfalls durch $C_{1-4}$-Alkyl substituiert sein kann; einen araliphatischen Rest mit 7—12 C-Atomen, wobei das aromatische Ringsystem gegebenenfalls durch Halogen, Nitro, Trifluormethyl, Cyano, $C_{1-4}$-Alkyl und/oder $C_{1-4}$-Alkoxy substituiert sein kann; einen aromatischen Rest mit 6—12 C-Atomen, der gegebenenfalls durch Halogen, Nitro, Trifluormethyl, Cyano, $C_{1-4}$-Alkyl und/oder $C_{1-4}$-Alkoxy substituiert sein kann, oder für einen heterocyclischen Rest mit 5—6 Ringatomen, wobei 1—3 Heteroatome im Ringsystem vorhanden sein können, steht und

$R^2$ für einen geraden oder verzweigten Alkylrest mit 1—6 C-Atomen, der gegebenenfalls durch $C_{1-4}$-Alkoxy, $C_{1-4}$-Alkylmercapto, $C_{1-4}$-Alkoxycarbonyl, Halogen, Cyan oder Nitro substituiert sein kann; einen Alkenylrest mit 3—6 C-Atomen; einen Alkinylrest mit 3—6 C-Atomen; einen cycloaliphatischen Rest mit 5—8 C-Atomen, der gegebenenfalls durch $C_{1-4}$-Alkyl oder $C_{1-4}$-Alkoxy substituiert sein kann; oder für einen araliphatischen Rest mit 7—12 C-Atomen, wobei das aromatische Ringsystem gegebenenfalls durch $C_{1-4}$-Alkyl, $C_{1-4}$-Alkoxy, $C_{1-4}$-Alkylmercapto, $C_{1-4}$-Alkoxycarbonyl, Halogen, Cyan und/oder Nitro substituiert sein kann, steht,

auf technisch einfache Weise in hohen Ausbeuten erhält, wenn man

(a) Carbamidsäure-acrylester der allgemeinen Formel

$$R^1—NH—CO—OR^3 \quad (II)$$

in welcher

$R^1$ die oben angegebene Bedeutung hat und

$R^3$ für Phenyl steht, welches gegebenenfalls durch $C_{1-4}$-Alkyl, $C_{1-4}$-Alkoxy, Halogen, Trifluormethyl, Cyano und/oder Nitro substituiert sein kann,

mit Kohlensäure-arylester-halogeniden der allgemeinen Formel

$$X—CO—OR^4 \quad (III)$$

in welcher

$R^4$ die oben für $R^3$ angegebene Bedeutung hat (wobei die Phenylreste gleich oder verschieden sein können) und

X für Halogen, z. B. Chlor, steht,

gegebenenfalls in Gegenwart eines Verdünnungsmittels, jedoch in Abwesenheit eines Säurebindemittels, bei Temperaturen zwischen 100 und 300°C umsetzt, wobei die Kohlensäure-arylesterhalogenide (III) bevorzugt in überstöchiometrischen Mengen eingesetzt werden,

(b) die hierbei erhaltenen, neuen N-substituierten Imidodicarbonsäure-diarylester der allgemeinen Formel

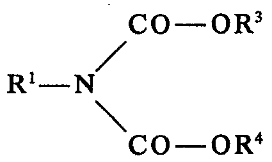

(IV)

in welcher

$R^1$, $R^3$ und $R^4$ die oben angegebene Bedeutung haben,

mit einem Isothiosemicarbazon der Formel

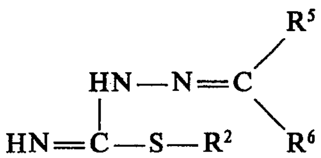

(V)

in welcher

$R^2$ die oben angegebene Bedeutung hat,

$R^5$ für Wasserstoff oder für Alkyl-, Cycloalkyl-, Aralkyl- oder Arylrest, die jeweils durch Halogen, Cyan, Nitro, Alkyl, Alkoxy oder Alkylmercapto substituiert sein können, steht, und

$R^6$ für Alkyl-, Cycloalkyl-, Aralkyl- oder Arylreste, die jeweils durch Halogen, Cyan, Nitro, Alkyl, Alkoxy oder Alkylmercapto substituiert sein können, steht, oder

$R^5$ und $R^6$, zusammen mit dem Alkyliden-C-Atom, für einen 5- bis 7-gliedrigen carbocyclischen Ring stehen,

bei Temperaturen zwischen 50 und 150°C umsetzt und

(c) die hierbei gebildeten, teilweise neuen 1-Alkylidenamino-1,3,5-triazin-2,4(1 H, 3 H)-dione der allgemeinen Formel

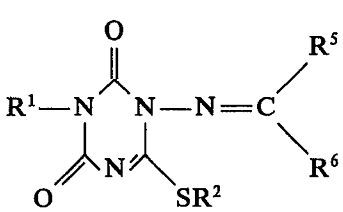

(VI)

in welcher

$R^1$, $R^2$, $R^5$ und $R^6$ die oben angegebene Bedeutung haben,

gegebenenfalls ohne Zwischenisolierung in an sich bekannter Weise in saurem Medium hydrolysiert.

Das erfindungsgemäße Verfahren weist eine Reihe von überraschenden Vorteilen auf: Die bei dem erfindungsgemäßen Verfahren als Ausgangsstoffe verwendeten Carbamidsäurearylester sind bekannt oder können nach bekannten Verfahren auf einfache Weise aus technisch leicht zugänglichen Vorprodukten in hoher Ausbeute hergestellt werden.

Ferner kann man insbesondere die Umsetzung nach Verfahrensstufe (b) ohne Verwendung von Lösungsmitteln in der Schmelze der Ausgangsprodukte durchführen. Hierbei werden keine weiteren Hilfsstoffe, wie z. B. organische Basen, benötigt.

Verwendet man in Verfahrensstufe (a) Neopentyl-carbamidsäure-phenylester und Kohlensäure-phenylester-chlorid sowie in Verfahrensstufe (b) zusätzlich Aceton-S-ethyl-isothiosemicarbazon als Ausgangsstoffe, so kann der Reaktionsablauf nach dem erfindungsgemäßen Verfahren durch das folgende Formelschema wiedergegeben werden:

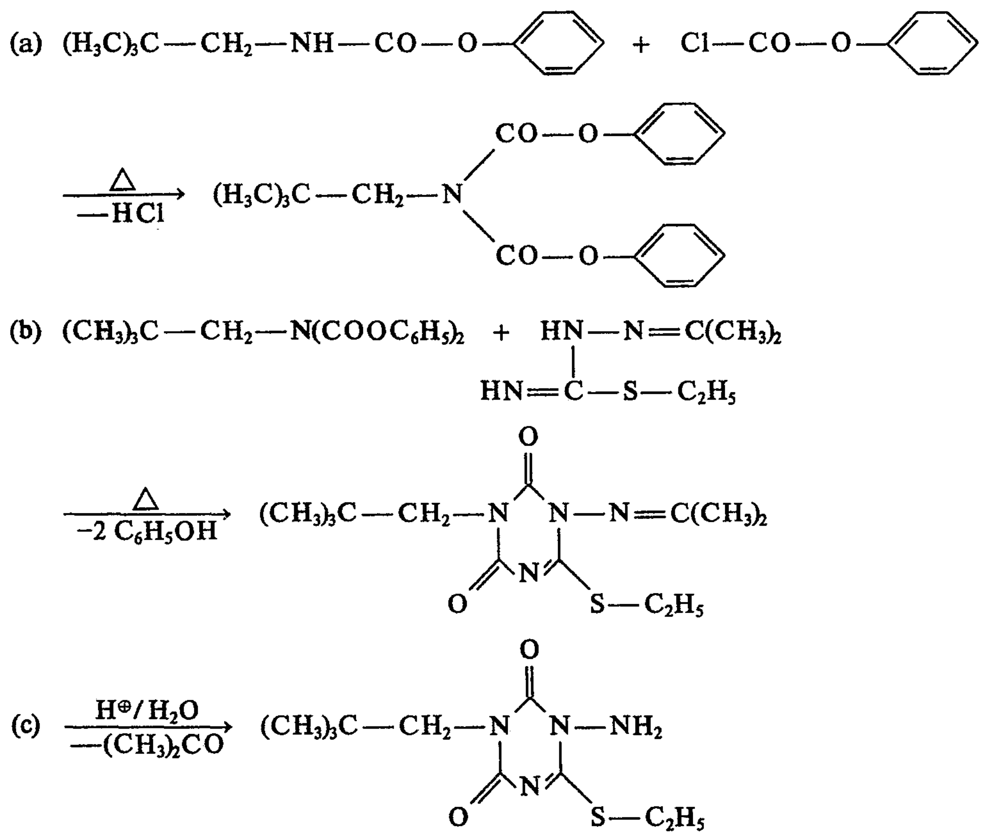


Die als Ausgangsstoffe verwendeten Carbamidsäure-arylester der Formel (II) sind bereits bekannt oder können nach bekannten Verfahren durch Addition von Isocyanaten an Phenole (vgl. Houben–Weyl, Methoden der organischen Chemie, 4. Auflage Bd. 8, S. 142 [1952]) oder durch Umsetzung von Kohlensäure-arylesterchloriden mit primären Aminen (vgl. Houben–Weyl, Methoden der organischen Chemie, 4. Auflage Bd. 138 [1952] sowie Herstellungsbeispiele) hergestellt werden. Die Ausgangsverbindungen der Formel (II) können ferner durch Umsetzung von Kohlensäure-diarylestern mit Aminen hergestellt werden (vgl. Houben–Weyl, Methoden der oragnischen Chemie, 4. Aufl. Bd. 8, S. 139 [1952]).

Die Kohlensäure-arylester-halogenide (III) sind ebenfalls bereits bekannt oder können nach bekannten Verfahren hergestellt werden. So kann man z. B. die Kohlensäure-phenylester-chloride in an sich bekannter Weise durch Phosgenierung von Phenolen herstellen (vgl. Houben–Weyl, Methoden der organischen Chemie, 4. Aufl. Bd. 8, S. 103 [1952]); die entsprechenden Kohlensäure-phenylesterfluoride können analog aus Phenolen und Difluorphosgen gewonnen werden (vgl. J. Chem. Soc. [London] 1948, S. 2183).

Die Reaktionstemperaturen können bei Verfahrensstufe (a) in einem größeren Bereich variiert werden. Im allgemeinen arbeitet man, wie oben angegeben, zwischen etwa 100 und 300°C, vorzugsweise zwischen etwa 170 und 250°C.

Weitere technische Einzelheiten zur Verfahrensstufe (a) können dem – hiermit gleichzeitig angemeldeten – Europäischen Patent Nr. 0 034 750 sowie den nachfolgenden Beispielen entnommen werden.

Die Zwischenisolierung der Imido-dicarbonsäure-diarylester (IV) erfolgt in einfacher Weise durch destillative Trennung des Reaktionsgemisches; feste, höher schmelzende Derivate lassen sich auch leicht durch Umkristallisieren reinigen.

Als Beispiele für die erfindungsgemäß nach Verfahrensstufe (a) herstellbaren N-substituierten Imido-dicarbonsäure-diarylester (IV) seien im einzelnen genannt: Gleiche oder gemischte Phenyl-, 2-Chlorphenyl-, 4-Chlorphenyl-, 4-Methylphenyl-, 4-Methoxyphenyl-, 4-Nitrophenyl-, 1-Naphthyl- oder 2-Naphthylester der Methyl-, Ethyl-, 2-Chlorethyl-, 2,2,2-Trifluorethyl-, Propyl-, Isopropyl-, tert.-Butyl-, sek.-Butyl-, Isobutyl-, Isopentyl-, Neopentyl-, 1-Ethylpropyl-, 1,2,2-Trimethylpropyl-, 2-Ethoxymethyl-, 2-Ethylmercaptoethyl-, ω-Cyanohexyl-, Allyl-, Propargyl-, Cyclopropylmethyl-, Cyclopentylmethyl-, Cyclohexylmethyl-, (2,5-Methano-cyclohexyl)-methyl-, Cycloheptylmethyl-, Cyclododekanylmethyl-, Adamantylmethyl-, 2-Furylmethyl-, 2-Pyranylmethyl-, 2-Pyridylmethyl-, 3-Pyridylmethyl-, 4-Pyridylmethyl-, 2-Methylpentyl-, 2-Ethylpentyl-, 2-Methylhexyl-, 2-Ethylhexyl-, Cyclopentyl-, Cyclohexyl-, 2-Methylcyclohexyl-, Benzyl-, 4-Chlorbenzyl-, 4-Nitrobenzyl-, Phenethyl-, 3-Chlorphenyl-, 4-Chlorphenyl-, 3,5-Dichlorphenyl-, 3,4-Dichlorphenyl-, 3-Trifluormethylphenyl-, 2-Chlor-4-nitro-phenyl-, 3-Methylphenyl-, 4-Methylphenyl-, 3-Methoxy-phenyl-, 1-Naphthyl-, 2-Furyl-, 4-Pyridyl-, 2-Thienyl-, 2-Benzthiazolyl- oder 2-Benzimidazolyl-imido-dicarbonsäure.

Die in Verfahrensstufe (b) weiterhin als Ausgangsstoffe zu verwendenden Isothiosemicarbazone der Formel (V) sind gleichfalls bekannt oder können nach bekannten Verfahren hergestellt werden, z. B. durch S-Alkylierung von Thiosemicarbazonen (vgl. Houben—Weyl, Methoden der organischen Chemie, 4. Aufl. Band 9, S. 912).

Als Beispiele für solche Isothiosemicarbazone (V) seien im einzelnen genannt: Aceton-S-methyl-, Aceton-S-ethyl-, Aceton-S-benzyl-, Aceton-S-carbomethoxymethyl-, Isobutyraldehyd-S-methyl-, Benzaldehyd-S-methyl-, Benzaldehyd-S-2-chlorethyl-, Aceton-S-allyl-, Aceton-S-propargyl-, Aceton-S-methoxymethyl-, Aceton-S-cyanmethyl-, Cyclopentanon-S-methyl-, Cyclohexanon-S-ethyl-, Cyclohexanon-S-carbethoxymethyl-, Cycloheptanon-S-ethyl-, Acetophenon-S-ethyl-, Benzophenon-S-methyl-, Butanon(2)-S-4-chlorbenzyl- oder Butanon(2)-S-ethyl-isothiosemicarbazon.

Die Verfahrensstufe (b), d. h. die Ringschlußreaktion (IV) + (V) ⟶ (VI), wird bevorzugt ohne Verdünnungsmittel in der Schmelze der Ausgangsprodukte durchgeführt. Es ist jedoch auch möglich, diese Umsetzung in Gegenwart eines inerten organischen Lösungsmittels als Verdünnungsmittel durchzuführen. In Frage kommen hierfür Kohlenwasserstoffe wie z. B. Toluol, chlorierte Kohlenwasserstoffe wie z. B. Chlorbenzol, oder Alkohole wie z. B. Isopropanol und sec.-Butanol.

Die Reaktionstemperaturen können bei Verfahrensstufe (b) ebenfalls in einem größeren Bereich variiert werden. Die Umsetzung wird im allgemeinen, wie oben angegeben, bei Temperaturen zwischen 50 und 150°C, vorzugsweise zwischen 70 und 120°C durchgeführt. Die Anwendung von erhöhtem Druck ist im allgemeinen nicht erforderlich.

Bei der Durchführung von Verfahrensstufe (b) setzt man auf 1 Mol des Imido-dicarbonsäure-diarylesters der Formel (IV) im allgemeinen 0,9—1,1 Mol des Isothiosemicarbazons der Formel (V) ein. Vorzugsweise werden die Komponenten im stöchiometrischen Molverhältnis 1 : 1 umgesetzt in der Weise, daß man eine der Komponenten aufschmilzt, die andere zugibt und so lange bei erhöhter Temperatur hält, bis die Reaktion beendet ist. Durch gaschromatografische Kontrolle kann der Reaktionsablauf leicht verfolgt werden.

Die hierbei als weitere Zwischenprodukte gebildeten 1-Alkylidenamino-1,3,5-triazin-2,4(1 H, 3 H)-dione der Formel (VI) können, falls gewünscht, ebenfalls zwischenisoliert werden. Die Aufarbeitung und Isolierung der Zwischenprodukte (VI) kann z. B. in der Weise erfolgen, daß man das bei der Cyclisierungsreaktion gebildete, gegebenenfalls substituierte Phenol bzw. Phenolgemisch im Vakuum abdestilliert und den Rückstand durch Destillation im Hochvakuum oder durch Umlösen, falls erforderlich, reinigt.

Die anschließende Hydrolyse (Verfahrensstufe (c)), zur Abspaltung des als Schutzgruppe dienenden Alkylidenrestes ($=CR^5R^6$), (VI) ⟶ (I), wird in an sich bekannter Weise in saurem Medium durchgeführt (vgl. z. B. DE-OS 2 254 200 bzw. US-PS 4 056 527). Es ist besonders zweckmäßig, die Zwischenprodukte (VI) in einem Alkohol wie beispielsweise Isopropanol zu lösen und bei Temperaturen zwischen etwa 40 und 70°C, gegebenenfalls bei vermindertem Druck, mit einer katalytischen Menge einer Säure, beispielsweise einer Mineralsäure wie Schwefelsäure oder einer organischen Sulfonsäure wie p-Toluolsulfonsäure, zu versetzen und die gebildete Carbonylverbindung der Formel $R^5-CO-R^6$ zusammen mit einem Teil des als Verdünnungsmittel eingesetzten Alkohols aus dem Reaktionsgemisch abzudestillieren. Die Isolierung der Endprodukte (I) erfolgt in bekannter Weise durch Auskristallisieren und Abfiltrieren; zur weiteren Reinigung können die Endprodukte (I) leicht umkristallisiert werden.

Die erfindungsgemäß herstellbaren 1-Amino-1,3,5-triazin-2,4(1 H, 3 H)-dione (I) sind großenteils bekannt und besitzen ausgezeichnete herbizide Eigenschaften (vgl. z. B. DE-OS 2 254 200; US-PS 4 056 527; ferner DK-PS 136 067).

Die in der zweiten Stufe des erfindungsgemäßen Verfahrens (Verfahrensstufe (b)) gebildeten, teilweise neuen 1-Alkylidenamino-1,3,5-triazin-2,4(1 H, 3 H)-dione (VI) sind nicht nur als Zwischenprodukte zur Herstellung der entsprechenden 1-Amino-Verbindungen (I) von Interesse, sondern besitzen darüber hinaus auch selbst ausgeprägte herbizide Wirksamkeit (bezüglich der bekannten Verbindungen der allgemeinen Formel (VI) vgl. ebenfalls DE-OS 2 254 200; US-PS 4 056 527; DK-PS 136 067).

Die neuen Verbindungen der allgemeinen Formel (VI) können in grundsätzlich gleicher Weise wie die vorbekannten Verbindungen formuliert und angewendet werden (vgl. auch nachfolgende Verwendungsbeispiele A und B).

Darüber hinaus ist es möglich, die in 6-Stellung der Verbindungen der allgemeinen Formel (I) und (VI) befindlichen $SR^2$-Reste durch Umsetzung mit primären oder sekundären Aminen gegen Alkylamino- bzw. Dialkylaminogruppen auszutauschen, wobei ebenfalls bekannte herbizide Wirkstoffe erhalten werden (vgl. ebenfalls die vorgenannten Druckschriften: DE-OS 2 254 200; US-PS 4 056 527; DK-PS 136 067).

Die nachfolgenden Herstellungs- und Verwendungsbeispiele sollen zur weiteren Erläuterung der Erfindung dienen.

## Herstellungsbeispiele

### Beispiel 1

#### a) Verfahrensstufe (a)

$$(CH_3)_2CH{-}CH_2{-}N(CO{-}O{-}C_6H_5)_2 \qquad (IV{-}1)$$

In einem mit Rührer, Gaseinleitungsrohr, Rückflußkühler und Tropftrichter ausgerüsteten 4-Halskolben bringt man unter Durchleiten eines Luft- oder Stickstoff-Stroms 600 ml (4,72 Mol) Kohlensäure-phenylester-chlorid zum Sieden. Bei einer Innentemperatur von 180—185 °C tropft man sodann innerhalb von 5 Stunden eine Lösung von 77,2 g (0,4 Mol) Isobutyl-carbamidsäure-phenylester (Schmelzpunkt 67 °C) in 300 ml Kohlensäure-phenylester-chlorid (2,36 Mol) gleichmäßig zu, wobei weiter Luft oder Stickstoff durch die Reaktionslösung geleitet wird, um den gebildeten Chlorwasserstoff rasch abzuführen. Man rührt noch 2 Stunden bei Siede-Temperatur nach, destilliert das überschüssige Kohlensäure-phenylester-chlorid bei einer Bad-Temperatur von 140 °C und einem Druck von 20 mbar ab und destilliert den Rückstand im Vakuum.

Man erhält 102,4 g N-Isobutyl-imido-dicarbonsäure-diphenylester mit einem Siedepunkt von 160 °C/0,1 mbar und einer Reinheit von 93,7 %. Nach dem Umlösen aus ca. 500 ml Petrolether, Absaugen bei bei −70 °C und Waschen mit tiefgekühltem Petrolether erhält man 88 g der genannten Verbindung mit einem Schmelzpunkt von 40 °C und einer Reinheit von 100 %, entsprechend 70 % d. Th.

#### b) Verfahrensstufe (b)

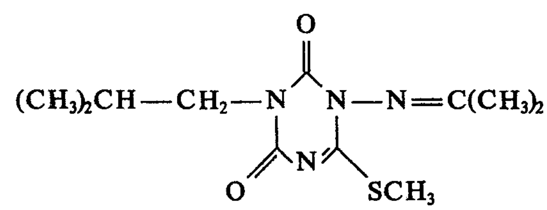

(VI−1), bekannt

34,6 g (0,11 Mol) N-Isobutyl-imido-dicarbonsäure-diphenylester (IV−1) und 16,0 g (0,11 Mol) Aceton-S-methyl-isothiosemicarbazon werden bei 50 °C aufgeschmolzen und in einem Ölbad von 100 °C 4 Stunden lang gerührt. Man destilliert das gebildete Phenol bei einem Druck von 18 mbar ab, wobei man die Bad-Temperatur bis auf 140 °C steigert. Der Rückstand (30,3 g) erstarrt; er wird mit 150 ml Cyclohexan aufgekocht, wobei 22,4 g reines 1-Isopropylidenamino-3-isobutyl-6-methylthio-1,3,5-triazin-2,4(1 H, 3 H)-dion (VI−1) vom Schmelzpunkt 125—127 °C ungelöst zurück bleiben. Aus dem Filtrat davon kristallisieren weitere 6,4 g (VI−1). Die Gesamtausbeute beträgt 28,8 g (97 % d. Th.). Die Verbindung (VI−1) kann destilliert werden: Siedepunkt 165 °C bei 0,38 mbar.

#### c) Verfahrensstufe (c)

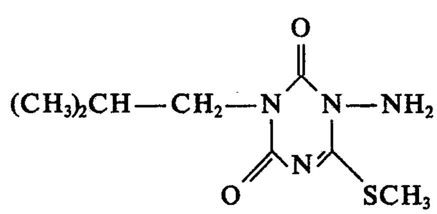

(I−1), bekannt

In einer Destillationsapparatur löst man 27,0 g (0,1 Mol) der Verbindung (VI−1) bei 60 °C in 200 ml Isopropanol und stellt einen Druck von 260—200 mbar ein, so daß das Lösungsmittel zu sieden beginnt und im absteigenden Kühler kondensiert. Die Innentemperatur beträgt dann 45—50 °C. Sodann tropft man innerhalb 15 Minuten eine Lösung von 0,4 ml konz. Schwefelsäure in 7 ml Wasser zu, wobei man während dieser Zeit ca. 70 ml Isopropanol, zusammen mit dem gebildeten Aceton, abdestilliert. Aus der verbleibenden Lösung kristallisieren bei 0 °C 14,5 g 1-Amino-3-isobutyl-6-methylthio-1,3,5-triazin-2,4(1 H, 3 H)-dion (I−1) vom Schmelzpunkt 167—169 °C aus; aus dem eingeengten Filtrat davon werden weitere 4,5 g erhalten. Die Gesamtausbeute von 19,0 g entspricht 83 % d. Th.

Beispiel 2

a) Verfahrensstufe (a)

$$(CH_3)_3C-CH_2-N(CO-O-C_6H_5)_2 \qquad (IV-2)$$

Eine Lösung von 331 g (1,6 Mol) Neopentyl-carbamidsäure-phenylester in 1000 g (6,39 Mol) Kohlensäure-phenylester-chlorid tropft man innerhalb 5 Stunden in 4000 g (25,56 Mol) siedendes Kohlensäure-phenylester-chlorid ein, durch das ein kräftiger Stickstoffstrom geleitet wird. Die Kopftemperatur am Rückflußkühler wird bei 80—90°C gehalten, so daß das in geringer Menge als Nebenprodukt gebildete Neopentylisocyanat mit dem Stickstoffstrom über Kopf destilliert und in einem nachgeschalteten absteigenden Kühler kondensiert werden kann. (Nach der Umsetzung mit Phenol zu Neopentylcarbamidsäure-phenylester wird es dann erneut der Reaktion zugeführt). Man rührt 4 Stunden unter weiterem Durchleiten von Stickstoff nach, destilliert sodann das überschüssige Kohlensäure-phenylester-chlorid bei einer Bad-Temperatur von 140°C und einem Druck von 20 mbar ab und destilliert den Rückstand aus einem Heizbad von 170°C, bis eine Siede-Temperatur von 150°C bei einem Druck von 0,6 mbar erreicht ist. Der Rückstand besteht aus 96,5%igem N-Neopentyl-imido-dicarbonsäure-diphenylester. Ausbeute: 489 g (90 % d. Th.). Eine aus Petrolether umkristallisierte Probe schmilzt bei 81 °C. Die Substanz ist destillierbar: Siedepunkt 156°C/0,02 mbar.

Der noch nicht bekannte, als Ausgangsverbindung verwendete Neopentyl-carbamidsäure-phenylester kann beispielsweise, ausgehend von Neopentylamin, folgendermaßen hergestellt werden:

Eine Lösung von 80 g (2 Mol) Natriumhydroxid und 176 g (2 Mol) 99%igem Neopentylamin in 3,4 Liter Wasser tropft man unter kräftigem Rühren in eine Lösung von 329 g (2,1 Mol) Kohlensäure-phenylester-chlorid in 1 Liter Toluol. Man hält durch Kühlung eine Innentemperatur von 10—20°C. Nach beendeter Reaktion trennt man die Phasen, wäscht die organische Phase mit Wasser, filtriert und dampft zur Trockne ein. Man erhält 408 g eines 97%igen Rohproduktes (95,6 % d. Th.) vom Schmelzpunkt 69—72 °C, für weitere Umsetzungen ausreichend rein. Nach dem Umlösen aus 2 Liter Petrolether erhält man 365 g vom Schmelzpunkt 77—78°C.

b) Verfahrensstufe (b)

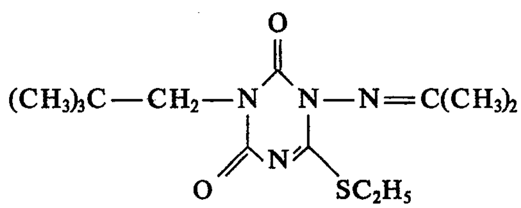

(VI−2), neu

Das Zwischenprodukt (VI−2), 1 - Isopropylidenamino - 6 - ethylthio - 3 - neopentyl - 1,3,5 - triazin-2,4(1 H, 3 H)-dion, kann analog zu Beispiel 1 b) hergestellt und isoliert werden; Schmelzpunkt 100—102°C, Siedepunkt 176°C/0,4 mbar.

c) Verfahrensstufe (c)

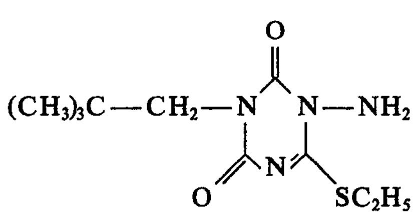

(I−2), bekannt

Die Verbindung (I−2), 1-Amino-6-ethylthio-3-neopentyl-1,3,5-triazin-2,4(1 H, 3 H)-dion, kann analog zu Beispiel 1 c) hergestellt werden; Schmelzpunkt 202—204°C.

Die Verbindung (I−2) kann jedoch auch ohne Zwischenisolierung von (VI−2) in folgender Weise hergestellt werden:

Eine Mischung von 65,4 g (0,2 Mol) N-Neopentyl-imido-dicarbonsäure-diphenylester (IV−2) und 31,8 g (0,2 Mol) Aceton-S-ethyl-isothiosemicarbazon (V−2) wird unter Stickstoff aufgeschmolzen und 5 Stunden bei 100°C gerührt. Danach wird das gebildete Phenol im Vakuum abdestilliert.

Der Rückstand, der im wesentlichen aus 1-Isopropylidenamino-6-ethylthio-3-neopentyl-1,3,5-triazin-2,4(1 H, 3 H)-dion (VI−2) besteht, wird in 200 ml Isopropanol gelöst. Zur hydrolytischen Abspaltung der Isopropyliden-Schutzgruppe gibt man 2,8 g p-Toluolsulfonsäure zu und tropft bei einer Temperatur von 60°C und einem Druck von 200—300 mbar innerhalb einer halben Stunde 14,4 ml Wasser zu. Das gebildete Aceton wird während der Reaktion zusammen mit etwa 100 ml Isopropanol

abdestilliert. Das auskristallisierte 1-Amino-6-ethylthio-3-neopentyl-1,3,5-triazin-2,4(1 H, 3 H)-dion (I−2) wird bei 0°C abgesaugt und mit Methanol gewaschen. Man erhält 38,2 g (I−2) vom Schmelzpunkt 202°C, entsprechend einer Ausbeute von 74% d. Th.

Analog zu Beispiel 1 a) können die folgenden Imido-dicarbonsäure-diarylester (IV) hergestellt werden:

| Beispiel Nr. | Strukturformel | Verbindung Nr. | Schmelzpunkt |
|---|---|---|---|
| 3 a), 4 a) | $CH_3—N(CO—O—C_6H_5)_2$ | (IV−3) | 102−105°C |
| 5 a) | $(CH_3)_2CH—N(CO—O—C_6H_5)_2$ | (IV−5) | 35−37°C |
| 2 a), 6 a) | $(CH_3)_3C—CH_2—N(CO—O—C_6H_5)_2$ | (IV−2) (vgl. Beispiel 2 a) | 81°C |
| 7 a), 8 a) | $CF_3—CH_2—N(CO—O—C_6H_5)_2$ | (IV−7) | 76°C |
| 9 a) | 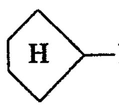 $—N(CO—O—C_6H_5)_2$ | (IV−9) | 53°C |
| 10 a) | 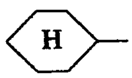 $—N(CO—O—C_6H_5)_2$ | (IV−10) | 85°C |

Analog zu Beispiel 1 b) können die folgenden 1-Alkylidenamino-1,3,5-triazin-2,4(1 H, 3 H)-dione (VI) hergestellt werden:

| Beispiel Nr. | Strukturformel | Verbindung Nr. | Schmelzpunkt (Siedepunkt) | bekannt/ neu |
|---|---|---|---|---|
| 3 b) | 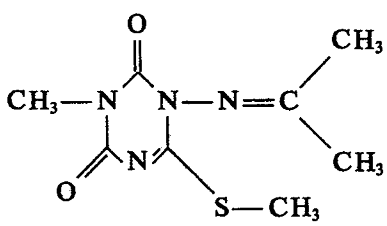 | (VI−3) | 130−131°C | bek. |
| 4 b) | 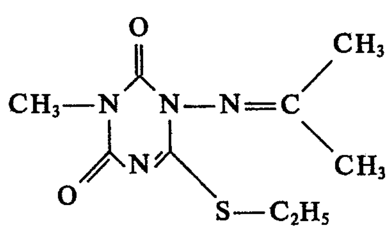 | (VI−4) | 121−122°C (173°C/ 0,5 mbar) | bek. |
| 5 b) | 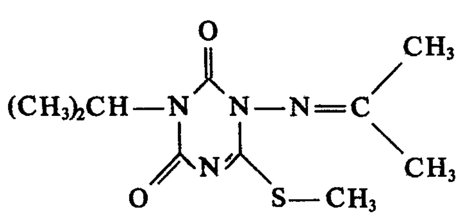 | (VI−5) | 110−112°C | bek. |

| Beispiel Nr. | Strukturformel | Verbindung Nr. | Schmelzpunkt (Siedepunkt) | bekannt/ neu |
|---|---|---|---|---|
| 6 b) | 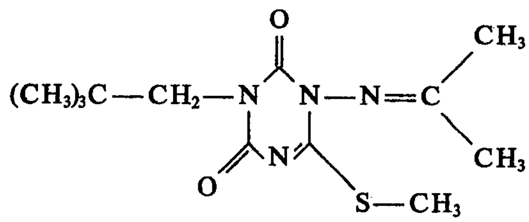 | (IV–6) | 122–124°C | neu |
| 7 b) | 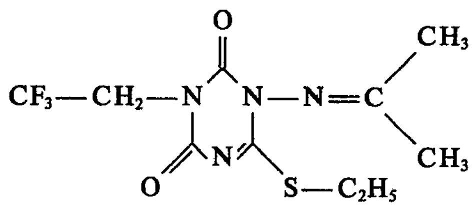 | (VI–7) | 142°C (145–150°C/ 0,3 mbar) | neu |
| 8 b) | 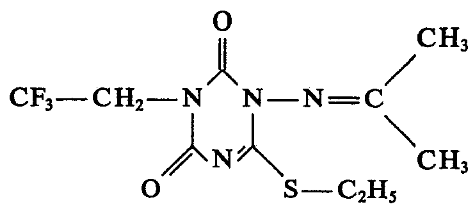 | (VI–8) | 112–114°C | neu |
| 9 b) | 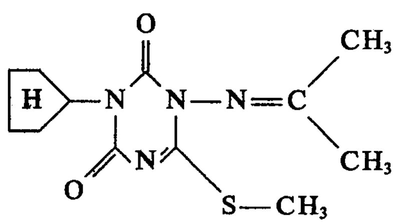 | (VI–9) | 107–109°C | neu |
| 10 b) | 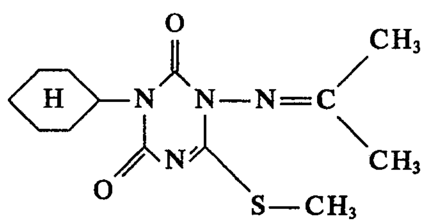 | (VI–10) | 111–112°C | bek. |

Analog zu Beispiel 1 c) oder 2 c) können die folgenden 1-Amino-1,3,5-triazin-2,4(1 H, 3 H)-dione (I) hergestellt werden:

| Beispiel Nr. | Strukturformel | Verbindung Nr. | Schmelzpunkt (Siedepunkt) | bekannt/ neu |
|---|---|---|---|---|
| 3 c) | 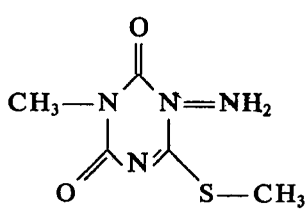 | (I–3) | 174–175°C | bek. |

(Fortsetzung)

| Beispiel Nr. | Strukturformel | Verbindung Nr. | Schmelzpunkt (Siedepunkt) | bekannt/ neu |
|---|---|---|---|---|
| 4 c) | 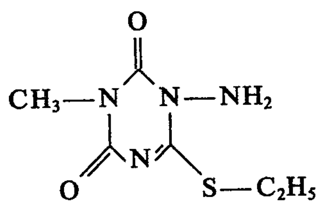 | (I–4) | 133–134°C | neu. |
| 5 c) | 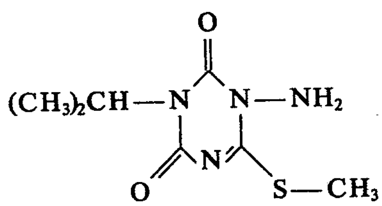 | (I–5) | 148–150°C | bek. |
| 6 c) | 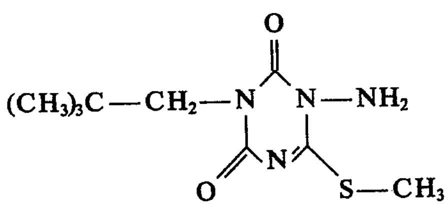 | (I–6) | 229–231°C | bek. |
| 7 c) | 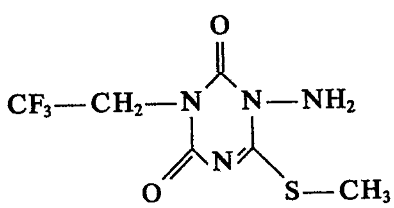 | (I–7) | 147–150°C | neu |
| 8 c) | 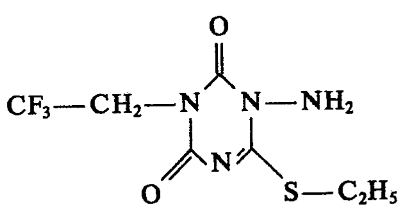 | (I–8) | 135–137°C | neu |
| 9 c) | 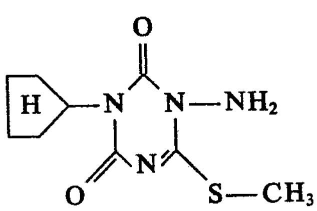 | (I–9) | 158–159°C | neu |
| 10 c) | 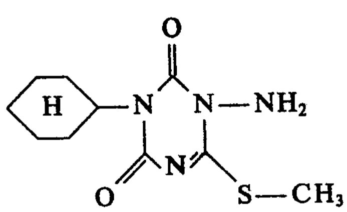 | (I–10) | 177–179°C | bek. |

## Verwendungsbeispiele

### Beispiel A

Pre-emergence-Test

Lösungsmittel: 5 Gewichtsteile Aceton
Emulgator: 1 Gewichtsteil Alkylarylpolyglycolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0% = keine Wirkung (wie unbehandelte Kontrolle)
100% = totale Vernichtung

In diesem Test zeigen die Verbindungen (VI–2), (VI–6), (VI–7), (VI–8), (VI–9), (I–4), (I–7), (I–8) und (I–9) eine ausgezeichnete Wirkung.

### Beispiel B

Post-emergence-Test

Lösungsmittel: 5 Gewichtsteile Aceton
Emulgator: 1 Gewichtsteil Alkylarylpolyglycolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5–15 cm haben, so daß die jeweils gewünschte Wirkstoffmenge pro Flächeneinheit ausgebracht wird. Die Konzentration der Spritzbrühe wird so gewählt, daß in 2000 l Wasser/ha die jeweils gewünschte Wirkstoffmenge ausgebracht wird. Nach drei Wochen wird der Schädigungsgrad der Pflanzen bonitiert in % Schädigung im Vergleich zur Entwicklung der unbehandelten Kontrolle. Es bedeuten:

0% = keine Wirkung (wie unbehandelte Kontrolle)
100% = totale Vernichtung

Auch in diesem Test zeigen die Verbindungen (VI–2), (VI–6), (VI–7), (VI–8), (VI–9), (I–4), (I–7), (I–8) und (I–9) eine hervorragende Wirkung.

## Patentansprüche

1. Verfahren zur Herstellung von 1-Amino-1,3,5-triazin-2,4(1 H, 3 H)-dionen der allgemeinen Formel

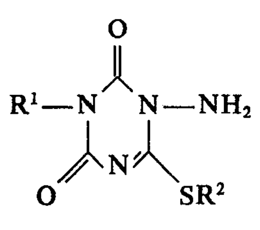

(I)

in welcher

$R^1$ für einen geradkettigen oder verzweigten Alkylrest mit 1–10 C-Atomen, der gegebenenfalls durch $C_{1-4}$-Alkoxy, $C_{1-4}$-Alkylmercapto, Halogen, Cyano oder Nitro substituiert sein kann; einen Alkenylrest mit 3—8 C-Atomen; einen Alkinylrest mit 3—8 C-Atomen; einen cycloaliphatischen Rest mit 5—8 C-Atomen, der gegebenenfalls durch $C_{1-4}$-Alkyl substituiert sein kann; einen araliphatischen Rest mit 7—12 C-Atomen, wobei das aromatische Ringsystem gegebenenfalls durch Halogen, Nitro, Trifluormethyl, Cyano, $C_{1-4}$-Alkyl und/oder $C_{1-4}$-Alkoxy substituiert sein kann; einen aromatischen Rest mit 6—12 C-Atomen, der gegebenenfalls durch Halogen, Nitro, Trifluormethyl, Cyano, $C_{1-4}$-Alkyl und/oder $C_{1-4}$- Alkoxy substituiert sein kann, oder für einen heterocyclischen Rest mit 5—6 Ringatomen, wobei 1—3 Heteroatome im Ringsystem vorhanden sein können, steht und

$R^2$ für einen geraden oder verzweigten Alkylrest mit 1—6 C-Atomen, der gegebenenfalls durch $C_{1-4}$-Alkoxy, $C_{1-4}$-Alkylmercapto, $C_{1-4}$-Alkoxycarbonyl, Halogen, Cyan oder Nitro substituiert sein kann; einen Alkenylrest mit 3—6 C-Atomen; einen Alkinylrest mit 3—6 C-Atomen; einen cycloaliphatischen Rest mit 5—8 Ring-C-Atomen, der gegebenenfalls durch $C_{1-4}$-Alkyl oder $C_{1-4}$-Alkoxy substituiert sein kann; oder für einen araliphatischen Rest mit 7—12 C-Atomen, wobei das aromatische Ringsystem gegebenenfalls durch $C_{1-4}$-Alkyl, $C_{1-4}$-Alkoxy, $C_{1-4}$-Alkylmercapto, $C_{1-4}$-Alkoxycarbonyl, Halogen, Cyan und/oder Nitro substituiert sein kann, steht,

dadurch gekennzeichnet, daß man

(a) Carbamidsäure-acrylester der allgemeinen Formel

$$R^1—NH—CO—OR^3 \quad (II)$$

in welcher

$R^1$ die oben angegebene Bedeutung hat und
$R^3$ für Phenyl steht, welches gegebenenfalls durch $C_{1-4}$-Alkyl, $C_{1-4}$-Alkoxy, Halogen, Trifluormethyl, Cyano und/oder Nitro substituiert sein kann,

mit Kohlensäure-arylester-halogeniden der allgemeinen Formel

$$X—CO—OR^4 \quad (III)$$

in welcher

$R^4$ die oben für $R^3$ angegebene Bedeutung hat (wobei die Phenylreste gleich oder verschieden sein können) und
X für Halogen, z. B. Chlor, steht,

gegebenenfalls in Gegenwart eines Verdünnungsmittels, jedoch in Abwesenheit eines Säurebindemittels, bei Temperaturen zwischen 100 und 300°C umsetzt, wobei die Kohlensäure-arylester-halogenide (III) bevorzugt in überstöchiometrischen Mengen eingesetzt werden,

(b) die hierbei erhaltenen, neuen N-substituierten Imidodicarbonsäure-diarylester der allgemeinen Formel

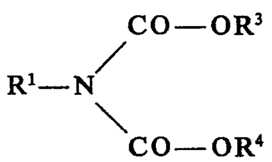

(IV)

in welcher

$R^1$, $R^3$ und $R^4$ die oben angegebene Bedeutung haben,

mit einem Isothiosemicarbazon der Formel

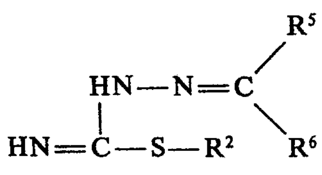

(V)

in welcher

$R^2$ die oben angegebene Bedeutung hat,

$R^5$ für Wasserstoff oder für Alkyl-, Cycloalkyl-, Aralkyl- oder Arylreste, die jeweils durch Halogen, Cyan, Nitro, Alkyl, Alkoxy oder Alkylmercapto substituiert sein können, steht, und

$R^6$ für Alkyl-, Cycloalkyl-, Aralkyl- oder Arylreste, die jeweils durch Halogen, Cyan, Nitro, Alkyl, Alkoxy oder Alkylmercapto substituiert sein können, steht, oder

$R^5$ und $R^6$, zusammen mit dem Alkyliden-C-Atom, für einen 5- bis 7-gliedrigen carbocyclischen Ring stehen,

bei Temperaturen zwischen 50 und 150°C umsetzt und

(c) die hierbei gebildeten, teilweise neuen 1-Alkylidenamino-1,3,5-triazin-2,4(1 H, 3 H)-dione der allgemeinen Formel

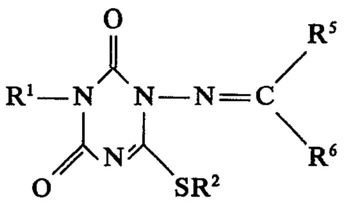

(VI)

in welcher

$R^1$, $R^2$, $R^5$ und $R^6$ die oben angegebene Bedeutung haben,

gegebenenfalls ohne Zwischenisolierung in an sich bekannter Weise in saurem Medium hydrolysiert.

2. Verfahren nach Anspruch 1, dadurch gekennzeichnet, daß man Verfahrensstufe (a) bei Temperaturen zwischen 170 und 250°C durchführt.

3. Verfahren nach Anspruch 1, dadurch gekennzeichnet, daß man Verfahrensstufe (b) bei Temperaturen zwischen 70 und 120°C durchführt.

4. Verfahren nach Anspruch 1, dadurch gekennzeichnet, daß man in Verfahrensstufe (b) auf 1 Mol des Imido-dicarbonsäure-diarylesters (IV) 0,9–1,1 Mol des Isothiosemicarbazons (V) einsetzt.

5. Verfahren nach Anspruch 1, dadurch gekennzeichnet, daß man als Carbamidsäure-arylester (II) Neopentyl-carbamidsäure-phenylester, als Kohlensäure-arylester-halogenid (III) Kohlensäure-phenylester-chlorid und als Isothiosemicarbazon (V) Aceton-S-ethyl-isothiosemicarbazon einsetzt.

**Claims**

1. Process for the preparation of 1-amino-1,3,5-triazine-2,4(1 H, 3 H)diones of the general formula

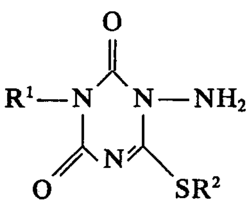

(I)

in which

$R^1$ represents a straight-chain or branched alkyl radical which has 1–10 C atoms and can optionally be substituted by $C_{1-4}$-alkoxy, $C_{1-4}$-alkylmercapto, halogen, cyano or nitro; an alkenyl radical with 3–8 C atoms; an alkinyl radical with 3–8 atoms; a cycloaliphatic radical which has 5–8 C atoms and can optionally be substituted by $C_{1-4}$-alkyl; an araliphatic radical with 7–12 C atoms, it being possible for the aromatic ring system optionally to be substituted by halogen, nitro, trifluoromethyl, cyano, $C_{1-4}$-alkyl and/or $C_{1-4}$-alkoxy; an aromatic radical which has 6–12 C atoms and can optionally be substituted by halogen, nitro, trifluoromethyl, cyano, $C_{1-4}$-alkyl and/or $C_{1-4}$-alkoxy, or a heterocyclic radical with 5–6 ring atoms, it being possible for 1–3 hetero-atoms to be present in the ring system, and

$R^2$ represents a straight or branched alkyl radical which has 1–6 C atoms and can optionally be substituted by $C_{1-4}$-alkoxy, $C_{1-4}$-alkylmercapto, $C_{1-4}$-alkoxycarbonyl, halogen, cyano or nitro; an alkenyl radical with 3–6 C atoms; an alkinyl radical with 3–6 C atoms; a cycloaliphatic radical which has 5–8 ring C atoms and can optionally be substituted by $C_{1-4}$-alkyl or $C_{1-4}$-alkoxy; or an araliphatic

radical with 7—12 C atoms, it being possible for the aromatic ring system optionally to be substituted by $C_{1-4}$-alkyl, $C_{1-4}$-alkoxy, $C_{1-4}$-alkylmercapto, $C_{1-4}$-alkoxycarbonyl, halogen, cyano and/or nitro,

characterised in that

(a) carbamic acid aryl esters of the general formula

$$R^1—NH—CO—OR^3 \quad (II)$$

in which

$R^1$ has the meaning given above and
$R^3$ represents phenyl which can optionally be substituted by $C_{1-4}$-alkyl, $C_{1-4}$-alkoxy, halogen, trifluormethyl, cyano und/or nitro,

are reacted with carbonic acid aryl ester halides of the general formula

$$X—CO—OR^4 \quad (III)$$

in which

$R^4$ has the meaning given above for $R^3$ (it being possible for the phenyl radicals to be identical or different) and
X represents halogen, for example chlorine,

if appropriate in the presence of a diluent, but in the absence of an acid-binding agent, at temperatures between 100 and 300°C, the carbonic acid aryl ester halides (III) preferably being employed in amounts greater than the stoichiometric amount,

(b) the new N-substituted imido-dicarboxylic acid diaryl esters thereby obtained, of the general formula

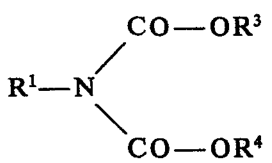
(IV)

in which

$R^1$, $R^3$ and $R^4$ have the meaning given above,

are reacted with an isothiosemicarbazone of the formula

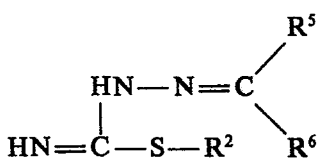
(V)

in which

$R^2$ has the meaning given above,
$R^5$ represents hydrogen or alkyl, cycloalkyl, aralkyl or aryl radicals which can each be substituted by halogen, cyano, nitro, alkyl, alkoxy or alkylmercapto, and
$R^6$ represents alkyl, cycloalkyl, aralkyl or aryl radicals, which can each be substituted by halogen, cyano, nitro, alkyl, alkoxy or alkylmercapto, or
$R^5$ und $R^6$, together with the alkylidene C atom, represent a 5- to 7-membered carbocyclic ring,

at temperatures between 50 and 150°C and

(c) the in some cases new 1-alkylidenamino-1,3,5-triazine-2,4(1 H, 3 H)diones thereby formed, of the general formula

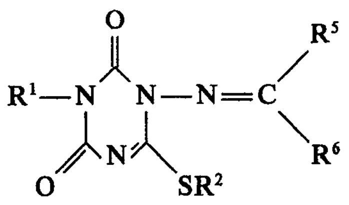

(VI)

in which

$R^1$, $R^2$, $R^5$ und $R^6$ have the meaning given above,

are hydrolysed in an acid medium in a manner known per se, if desired without being intermediately isolated.

2. Process according to Claim 1, characterised in that process stage (a) is carried out at temperatures between 170 and 250°C.

3. Process according to Claim 1, characterised in that process stage (b) is carried out at temperatures between 70 and 120°C.

4. Process according to Claim 1, characterised in that in process stage (b) 0.9—1.1 moles of the isothiosemicarbazone (V) are employed per mole of the imido-dicarboxylic acid diaryl ester (IV).

5. Process according to Claim 1, characterised in that neopentylcarbamic acid phenyl ester is employed as the carbamic acid aryl ester (II), carbonic acid phenyl ester chloride is employed as the carbonic acid aryl ester halide (III) and acetone S-ethyl-isothiosemicarbazone is employed as the isothiosemicarbazone (V).

**Revendications**

1. Procédé de préparation de 1-amino-1,3,5-triazine-2,4(1 H, 3 H)-diones de formule générale:

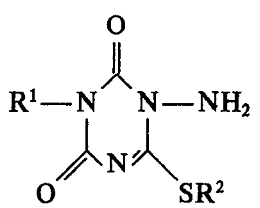

(I)

dans laquelle

$R^1$ représente un groupe alkyle à chaîne droite ou ramifiée contenant 1 à 10 atomes de carbone et pouvant éventuellement être substitué par un groupe alcoxy en $C_1$—$C_4$, par un groupe alkyl(en $C_1$—$C_4$)mercapto, par un atome d'halogène, par un groupe cyano ou par un groupe nitro; un groupe alcényle contenant 3 à 8 atomes de carbone; un groupe alcinyle contenant 3 à 8 atomes de carbone; un groupe cycloaliphatique contenant 5 à 8 atomes de carbone et pouvant éventuellement être substitué par un groupe alkyle en $C_1$—$C_4$; un groupe araliphatique contenant 7 à 12 atomes de carbone, le système à noyau aromatique pouvant éventuellement être substitué par un atome d'halogène, par un groupe nitro, par un groupe trifluorométhyle, par un groupe cyano, par un groupe alkyle en $C_1$—$C_4$ et/ou par un groupe alcoxy en $C_1$—$C_4$; un groupe aromatique contenant 6 à 12 atomes de carbone et pouvant éventuellement être substitue par un atome d'halogène, par un groupe nitro, par un groupe trifluorométhyle, par un groupe cyano, par un groupe alkyle en $C_1$—$C_4$ et/ou par un groupe alcoxy en $C_1$—$C_4$; ou un groupe hétérocyclique contenant 5 à 6 atomes cycliques, 1—3 hétéroatomes pouvant être présents dans le système cyclique, et

$R^2$ représente un groupe alkyle à chaîne droite ou ramifiée contenant 1 à 6 atomes de carbone et pouvant éventuellement être substitué par un groupe alcoxy en $C_1$—$C_4$, par un groupe alkyl(en $C_1$—$C_4$)mercapto, par un groupe alcoxy(en $C_1$—$C_4$)carbonyle, par un atome d'halogène, par un groupe cyano ou par un groupe nitro; un groupe alcényle contenant 3 à 6 atomes de carbone; un groupe alcinyle contenant 3 à 6 atomes de carbone; un groupe cycloaliphatique contenant 5 à 8 atomes de carbone cyclique et pouvant éventuellement être substitué par un groupe alkyle en $C_1$—$_4$ ou par un groupe alcoxy en $C_1$—$C_4$; ou un groupe araliphatique contenant 7 à 12 atomes de carbone, le système à noyau aromatique pouvant éventuellement être substitué par un groupe alkyle en $C_1$—$C_4$, par un groupe alcoxy en $C_1$—$C_4$, par un groupe alkyl(en $C_1$—$C_4$)mercapto, par un groupe alcoxy(en $C_1$—$C_4$)carbonyle, par un atome d'halogène, par un groupe cyano et/ou par un groupe nitro,

caractérisé en ce que:

(a) on fait réagir des esters aryliques d'acidescarbamiques de formule générale:

$R^1—NH—CO—OR^3$ (II)

dans laquelle

$R^1$ a la signification indiquée ci-dessus, et
$R^3$ représente un groupe phényle pouvant éventuellement être substitué par un groupe alkyle en $C_1—C_4$, par un groupe alcoxy en $C_1—C_4$, par un atome d'halogène, par un groupe trifluorométhyle, par un groupe cyano et/ou par un groupe nitro,

avec des halogénures d'esters aryliques d'acide carbonique de formule générale:

$X—CO—OR^4$ (III)

dans laquelle

$R^4$ a la signification indiquée ci-dessus pour $R^3$ (les groupes phényle pouvant être identiques ou différents) et
X représente un atome d'halogène, par exemple, un atome de chlore,

éventuellement en présence d'un diluant, mais en absence d'un agent fixateur d'acide, à des températures comprises entre 100 et 300°C, les halogénures d'esters aryliques d'acide carbonique (III) étant utilisés, de préférence, en quantités surstoechiométriques,

(b) on fait réagir les nouveaux esters diaryliques d'acides imido-dicarboxyliques N-substitués ainsi obtenus de formule générale:

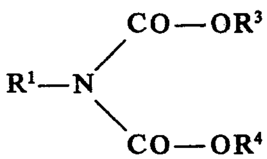
(IV)

dans laquelle

$R^1$, $R^3$ et $R^4$ ont les significations indiquées ci-dessus,

avec une isothiosemicarbazone de formule:

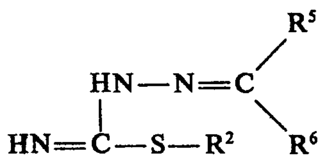
(V)

dans laquelle

$R^2$ a la signification indiquée ci-dessus,
$R^5$ représente un atome d'hydrogène, ou un groupe alkyle, un groupe cycloalkyle, un groupe aralkyle ou un groupe aryle pouvant être substitués chacun par un atome d'halogène, par un groupe cyano, par un groupe nitro, par un groupe alkyle, par un groupe alcoxy ou par un groupe alkylmercapto, et
$R^6$ représente un groupe alkyle, un groupe cycloalkyle, un groupe aralkyle ou un groupe aryle pouvant être substitués chacun par un atome d'halogène, par un groupe cyano, par un groupe nitro, par un groupe alkyle, par un groupe alcoxy ou par un groupe alkylmercapto, ou
$R^5$ et $R^6$, ensemble avec l'atome de carbone du groupe alkylidène, représentent un noyau carbocyclique pentagonal à heptagonal,

à des températures comprises entre 50 et 150°C, et

(c) on hydrolyse les 1-alkylidène-amino-1,3,5-triazine-2,4(1 H, 3 H)-diones partiellement nouvelles ainsi formées de formule générale:

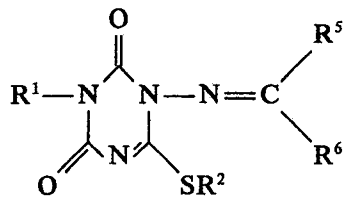

(VI)

dans laquelle

$R^1$, $R^2$, $R^5$ et $R^6$ ont les significations indiquées ci-dessus,

éventuellement sans isolation intermédiaire, de façon connue en soi, en milieu acide.

2. Procédé suivant la revendication 1, caractérisé en ce qu'on effectue l'étape opératoire (a) à des températures comprises entre 170 et 250°C.

3. Procédé suivant la revendication 1, caractérisé en ce qu'on effectue l'étape opératoire (b) à des températures comprises entre 70 et 120°C.

4. Procédé suivant la revendication 1, caractérisé en ce que, lors de l'étape opératoire (b), pour 1 mole de l'ester diarylique d'acide imidodicarboxylique (IV), on utilise 0,9—1,1 mole de l'isothiosemicarbazone (V).

5. Procédé suivant la revendication 1, caractérisé en ce que, comme ester arylique d'acide carbamique (II), on utilise l'ester phénylique d'acide néopentyl-carbamique; comme halogénure d'ester arylique d'acide carbonique (III), on utilise le chlorure d'ester phénylique d'acide carbonique; et, comme isothiosemicarbazone (V), on utilise l'acétone-S-éthyl-isothiosemicarbazone.